# EUROPEAN PATENT APPLICATION

(11) **EP 4 520 385 A1**
(43) Date of publication of application: **12.03.2025**
(21) Application number: 22946777.4
(22) Date of filing: 14.06.2022
(51) Int. Cl.: A61M 39/02, A61M 39/04, A61M 39/06

(54) **VALVE AND MEDICAL INSTRUMENT PROVIDED WITH SAME**

(71) Applicant: Togo Medikit Co., Ltd., Tokyo 113-0034 (JP)
(72) Inventor: MORINAGA, Noriaki, Tokyo 113-0034 (JP); ANDO, Hirokazu, Tokyo 113-0034 (JP)
(74) Representative: Herrero & Asociados, S.L.
(86) International application number: PCT/JP2022/023829
(87) International publication number: WO 2023/242957

(57) **Abstract**

[Problem] To provide: a valve that makes it possible to reliably balance an increase in sealing performance (prevention of air intake) with an increase in operability (slide characteristic) through use of such a structure that lubricant does not drip; and a medical instrument provided with the same. [Solution] Provided is hemostatic valve 1 which is bult into a valve holding part 24 inside a sheath hub 21b (valve housing) of a sheath 21 and into which a tubular introduction member such as a dilator and a catheter is inserted. The hemostatic valve 1 is provided with: a first partition wall 2 on a proximal side; a second partition wall 3 on a distal end side that is provided adjacent to the first partition wall 2; and a lubricant storing part 5 that is provided between the first partition wall 2 and the second partition wall 3 and can store lubricant. The lubricant storing part 5 is formed from a flat cylindrical recess formed on an inside surface of the first partition wall 2 in a center axis direction.

## Description

### TECHNICAL FIELD

The present disclosure relates to a valve and a medical device provided with the same. More specifically, the present disclosure relates to a valve through which a tubular introduction member such as a dilator, a catheter, or an endoscopic tube is inserted and to a medical device provided with the same.

### BACKGROUND

Conventionally, this type of valve, for example, disclosed in Patent Document 1, etc., is known.

The valve (hemostasis valve) disclosed in Patent Document 1 includes a proximal valve gasket (the first partition wall in a proximal side) and a distal valve gasket (second partition wall in a distal side), in which the outlet face of the proximal valve gasket is in contact with the inlet face of the distal valve gasket. The proximal valve gasket and the distal valve gasket are held firmly in a valve housing (see paragraph [0040] and Figures 1 to 4, etc., of Patent Document 1). The hemostasis valve including (the proximal valve gasket and the distal valve gasket) is made of a flexible and highly elastic polymeric material so that a cylindrical medical device (tubular introduction member) such as a long and thin dilator or catheter with a non-constant diameter can easily and repeatedly pass (be inserted) through the hemostasis valve (see paragraph [0040] and Figure 10, etc. of Patent Document 1).

As a medical device, hemostasis cannula assembly (introducer sheath) provided with such a hemostasis valve is used, for example, when a cardiac catheter is inserted from a blood vessel (vena cava) into the left atrium.

### DOCUMENT IN THE EXISTING ART

### Patent Document

Patent Document 1: JP 4940526 B

### SUMMARY

### The technical problem solved by the disclosure

A hemostasis valve must remain sealed even when no cylindrical medical device (tubular introduction member) is inserted through the valve.

However, when this is attempted, the resistance of the cylindrical medical device to be inserted through the hemostasis valve inevitably increases, resulting in poor operability (slidability) for insertion and removal of the cylindrical medical device. If the resistance of the cylindrical medical device to be inserted through the hemostasis valve is increased, the hemostasis valve may come off during insertion and removal of the cylindrical medical device.

In addition, a patient with spontaneous breathing may have negative pressure in the left atrium, especially during insertion and removal of the cylindrical medical device (tubular introduction member), which may increase the risk of air entering from the hemostasis valve (risk of air draw-in) if the pressure in the left atrium is negative.

However, when the sealing performance of the hemostasis valve is improved to prevent this risk, the resistance of the cylindrical medical device to be inserted through the hemostasis valve increases, resulting in poor operability (slidability) for insertion and removal of the cylindrical medical device.

Therefore, in the past, lubricant such as lubricating oil was applied to the surface of the hemostasis valve to achieve a balance between improved sealing performance (prevention of air draw-in) and improved operability (slidability).

However, if the lubricant is only applied to the surface of the valve, the lubricant will drip down due to gravity during the period from manufacture to use of the valve, or while a medical device provided with the valve, such as the hemostasis cannula assembly (introducer sheath), is being used. As the result, the operability (slidability) was reduced, making it difficult to achieve a balance between improved sealing performance (prevention of air draw-in) and improved operability (slidability), and there remained room for improvement from this perspective.

Therefore, an objective of the present disclosure is to provide a valve and a medical device provided with the same that has a structure that does not allow lubricant to drip down and that reliably enables a balance between improved sealing performance (prevention of air draw-in) and improved operability (slidability).

### Solution for solving the technical problem

In order to achieve the above-mentioned objective, the configuration of the valve according to the present disclosure is:
(1) A valve through which a tubular introduction member is inserted, including:
   a first partition wall in a proximal side;
   a second partition wall in a distal side that is provided adjacently to the first partition wall; and
   a lubricant storage that is provided between the first partition wall and the second partition wall, the lubricant storage having capability of storing lubricant.

In the present disclosure, "adjacent" is not only the situation where the first partition wall and the second partition wall are in direct contact to each other side by side but also the situation where lubricant is sealed in proximity between the first partition wall and the second partition wall through a sealing member such as an O-ring.

In the present disclosure, the first partition wall and the second partition wall are preferably made of a flexible and highly elastic polymeric material such as natural rubber, isoprene rubber, silicone rubber, or butadiene rubber.

A liquid lubricant can be used as "lubricant" in the present disclosure, for example. Examples of the liquid lubricant include a lubricating oil, more specifically, a silicone oil. A semi-solid lubricant such as a grease or a compound can also be used as "lubricant". A solid lubricant such as molybdenum disulfide and graphite added to a liquid lubricant such as silicone oil can also be used as "lubricant".

The above-mentioned configuration (1) of the valve of the present disclosure has the following effects.

Since a lubricant storage that has capability of storing lubricant is provided between the first partition wall and the second partition wall, the lubricant will never drip down due to gravity during the period from manufacture to use of the valve or while a medical device provided with the valve, such as a hemostasis cannula assembly (introducer sheath), is being used. Since the lubricant does not drip in this manner, the amount of lubricant can be increased.

Therefore, the above-mentioned configuration (1) of the valve of the present disclosure has a structure that does not allow lubricant to drip down and that reliably enables a balance between improved sealing performance (prevention of air draw-in) and improved operability (slidability).

The above-mentioned configuration (1) of the valve according to the present disclosure preferably has the following configurations (2) to (7).

(2) In the above-mentioned configuration (1), a groove for an adhesive is formed on the outer circumferential face of the first partition wall and/or the second partition wall.

The first partition wall and the second partition wall are embedded into the valve container in the valve housing of the medical device after adhesive is applied to the outer circumferential face. Since the outer diameters of the first partition wall and the second partition wall are larger than the inner diameter of the valve container, most of the adhesive may be scraped off during the embedding. This may reduce the adhesive force between the first partition wall and/or the second partition wall and the valve container.

However, according to the above-mentioned preferred configuration (2), when the adhesive is applied, the adhesive is filled into the groove for the adhesive formed on the outer circumferential face of the first partition wall and/or the second partition wall, and the adhesive will never be scraped off during the embedding. As the result, the decrease in adhesive force between the first partition wall and/or the second partition wall and the valve container can be minimized.

(3) In the above-mentioned configuration (1), a lubricant reservoir is formed on at least the outer (outside) face of the first partition wall and/or the second partition wall in the central axial direction. According to the above-mentioned preferred configuration (3), even when lubricant is applied on at least the outer (outside) face of the first partition wall and/or the second partition wall in the central axial direction, the lubricant remains in the lubricant reservoir. As the result, the lubricant is less likely to drip down due to gravity during the period of manufacture to use of the valve or while a medical device such as a hemostasis cannula assembly (introducer sheath) is being used. Therefore, the lubricant applied to at least the outer side of the first partition wall and/or the second partition wall in the central axial direction also helps to further improve the operability (slidability) and sealing performance (prevention of air draw-in) when a tubular introduction member such as a dilator, a catheter, or an endoscopic tube is inserted and removed.

(4) In the above-mentioned configuration (1), a position fixing hole is formed on the outer edge of the outer face of the first partition wall and/or the second partition wall in the central axial direction. According to the above-mentioned preferred configuration (4), the first partition wall and/or the second partition wall can avoid shifting (moving) around the central axis. As the result, the appropriate relative position of the first partition wall and the second partition wall around the central axis can be secured, which can further improve operability (slidability) and maintain sealing performance when a tubular introduction member such as a dilator, a catheter, or an endoscopic tube is inserted and removed.

(5) In the above-mentioned configuration (1), a tub-shaped concave with a flat bottom is formed on the outer face of the second partition wall in the central axial direction. According to the above-mentioned preferred configuration (5), the valve portion of the second partition wall can be easily opened.

(6) In the above-mentioned configuration (1), a first and a second conical concaves are formed on the outer face of the first partition wall and the second partition wall in the central axial direction, respectively, and the distance between the inner ends of the first and the second conical concaves is set at 1.2 to 10.0 mm. According to the above-mentioned preferred configuration (6), it is possible to improve sealing performance by distance to further ensure that air is not drawn in.

(7) In the above-mentioned configuration (1), the lubricant storage includes at least a third conical concave formed on the inner (inside) face of the second partition wall in the central axial direction. According to the above-mentioned preferred configuration (7), it is possible to prevent, for example, a pigtail- or ring-shaped catheter from not advancing straight and from straying into the lubricant storage when such a catheter is inserted.

The medical device of the present disclosure includes:
(8) Any of the above-mentioned configurations (1) to (7) of the valve.

The above-mentioned configuration (8) of the medical device of the present disclosure is provided with the valve of the present disclosure so that the action and effect produced from the above-mentioned valve can be exerted.

### Technical effect

The present disclosure has a structure that does not allow lubricant to drip down and that reliably enables a balance between improved sealing performance (prevention of air draw-in) and improved operability (slidability).

### BRIEF DESCRIPTION OF THE DRAWINGS

FIG. 1 is a perspective view illustrating the external configuration of a medical device in one embodiment of the present disclosure.
FIG, 2 is a perspective view illustrating the external configuration of an introducer sheath, a component of a medical device in one embodiment of the present disclosure.
FIGs. 3A, 3B, and 3C are diagrams illustrating the external configuration of the sheath hub (valve housing), which is a component of the introducer sheath. (FIG. 3A is the distal end view, FIG. 3B is the side view, and FIG. 3C is the proximal end view).
FIG. 4 is a longitudinal cross-sectional view illustrating the internal configuration of the sheath hub (valve housing), which is a component of the introducer sheath.
FIG. 5 is a longitudinal cross-sectional view illustrating the configuration of a hemostasis valve in one embodiment of the present disclosure.
FIG. 6 is a diagram illustrating the first partition wall in a proximal side, which is a component of the hemostasis valve in one embodiment of the present disclosure, viewed from several directions.
FIGs. 7A and 7B are enlarged perspective views of the first partition wall in a proximal side, which is a component of the hemostasis valve in one embodiment of the present invention. (FIG. 7A is a view from the distal side, and FIG. 7B is a view from the proximal side).
FIG. 8 is a diagram illustrating another configuration of the first partition wall in a proximal side, which is a component of the hemostasis valve in one embodiment of the present disclosure, viewed from several directions.
FIGs. 9A and 9B are enlarged views of another configuration of the first partition wall in a proximal side, which is a component of the hemostasis valve in one embodiment of the present disclosure (FIG. 9A is the distal end view, and FIG. 9B is the proximal end view).
FIGs. 10A, 10B, and 10C are longitudinal cross-sectional views of another configuration of the hemostasis valve in one embodiment of the present disclosure, (in which the dimensions of the tub-shaped concaves in the second partition wall in a distal side are different in FIGs. 10A, 10B, and 10C).
FIG. 11 is a longitudinal cross-sectional view illustrating another configuration of the hemostasis valve in one embodiment of the present disclosure.
FIG. 12 is a longitudinal cross-sectional view illustrating another configuration of the hemostasis valve in one embodiment of the present disclosure.

### DETAILED DESCRIPTION OF THE EMBODIMENTS

The invention will be described more specifically below using suitable embodiments. However, the embodiments are merely examples embodying the present disclosure, which does not limit the present disclosure.

### Configuration of medical device

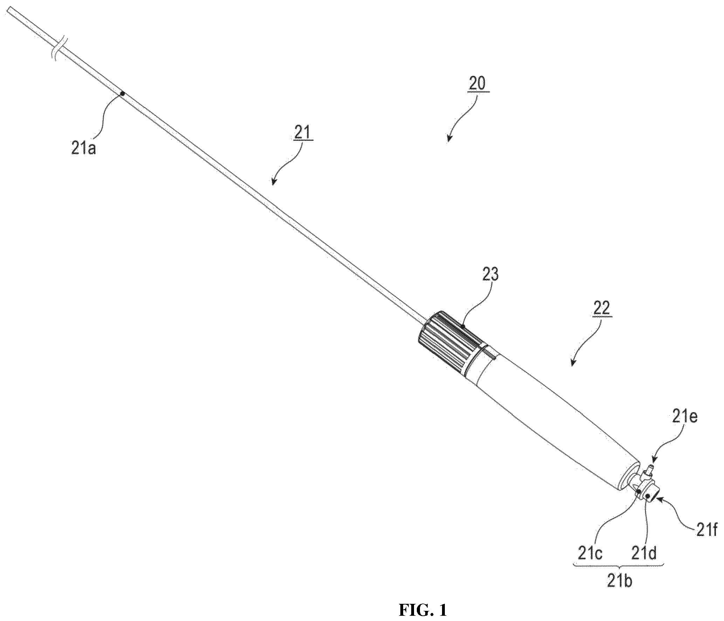

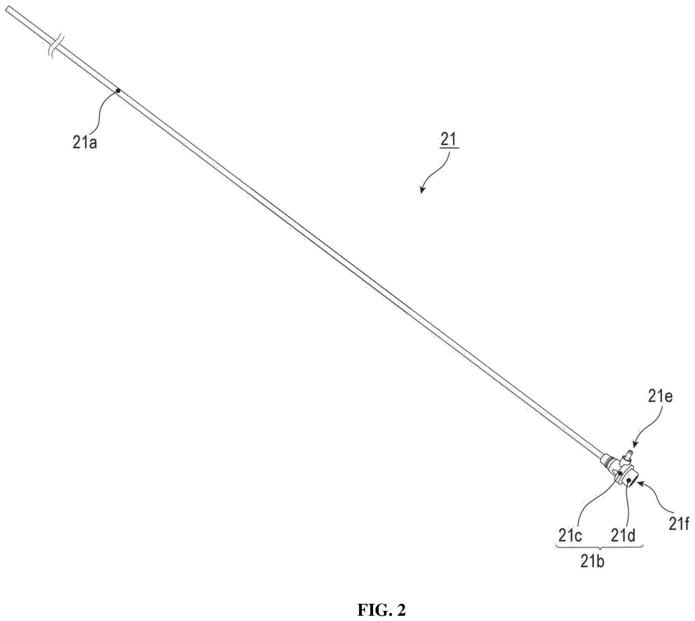

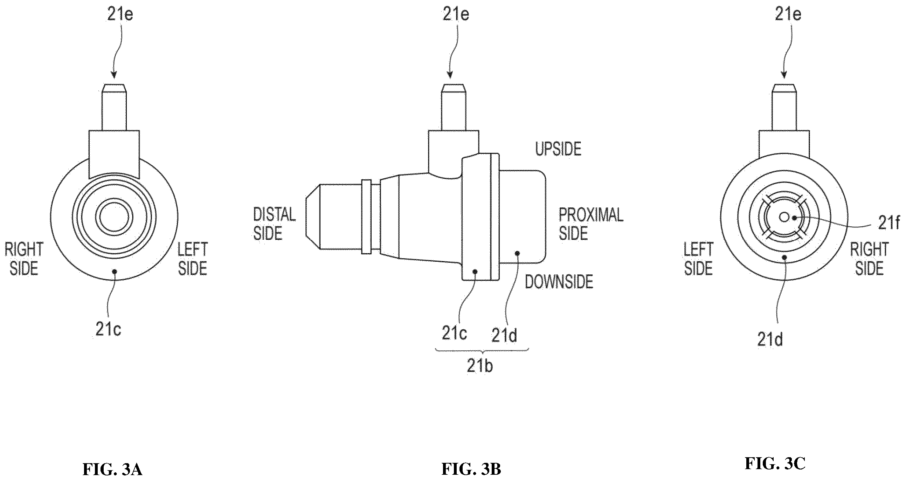

First, the configuration of the medical device provided with hemostasis valve in one embodiment of the present disclosure will be described with reference to FIGs. 1 to 4, using the case where a tubing member is an introducer sheath.

The medical device 20 shown in FIG. 1 is a tip-movable sheath that is provided with an introducer sheath (hereinafter simply referred to as a "sheath") 21 and a handle 22. The sheath 21 is formed of a flexible tube member. The handle 22 controls the orientation of the tip of the sheath 21. As shown in FIGs. 1 to 4, the sheath 21 is provided with a sheath tube 21a, a sheath hub (valve housing) 21b, a side port 21e, and a hemostasis valve 1 built in the sheath hub 21b. The sheath hub 21b is provided at the base end of the sheath tube 21a. The side port 21e is formed integrally with the sheath hub 21b and connected to the interior of the sheath tube 21a. The sheath 21 is used integrally with a dilator (not shown), for example, when a cardiac catheter is inserted from a blood vessel (vena cava) into the left atrium. At the back end of the sheath hub 21b is an insertion port 21f for inserting a dilator, catheter, etc.

A three-way stopcock (not shown) is attached to the side port 21e through a side tube (not shown). In order to prevent air from entering a blood vessel, heparin-saline solution (saline) is injected into the sheath tube 21a through the three-way stopcock (to replace air with saline) before the sheath tube 21a is inserted into the blood vessel. The three-way stopcock is also used to aspirate air that entered a blood vessel when the dilator was withdrawn or when another combination device was inserted into or out of the sheath tube 21a, after the sheath tube 21a was inserted into the blood vessel. Furthermore, a medical solution is also injected through the three-way stopcock.

The material of the sheath tube 21a is preferably a biocompatible synthetic resin selected from polyether block amide, polyamide (nylon 11), polytetrafluoroethylene, etc. The material of the sheath hub 21b is preferably a hard material such as a hard resin. Examples of the hard resin include polyethylene, polypropylene, polyamide, polycarbonate, and polystyrene.

The handle 22 is provided with a rotating operation portion (dial) 23 at the tip so that the direction of the tip of the sheath tube 21a can be controlled by rotating the rotating operation part (dial) 23. The specific configuration and the operation method of the handle 22 are disclosed in detail in JP 6847542 B and JP 6967644 B, which are used as references to omit duplication of explanation.

### Configuration of hemostasis valve

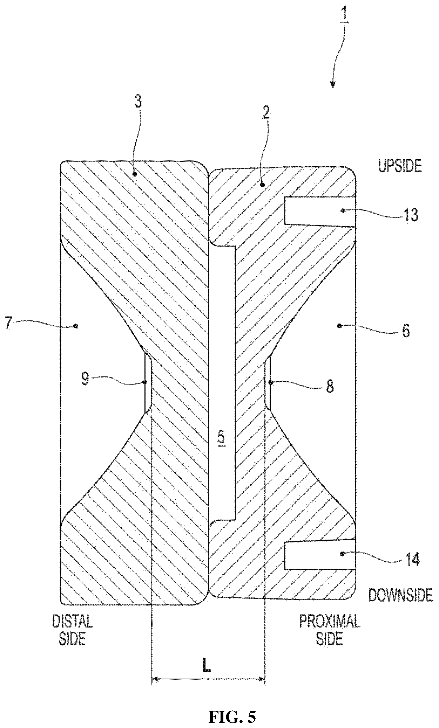

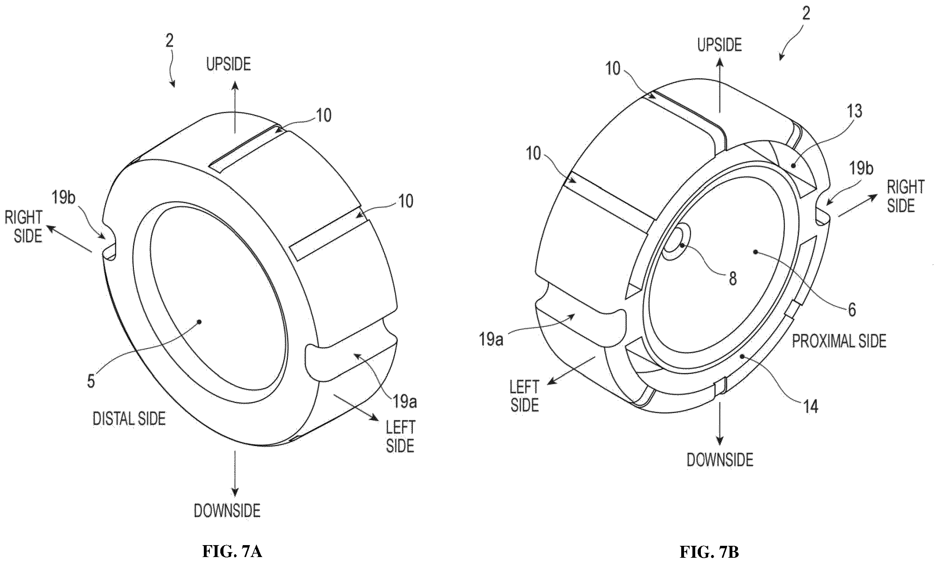

The specific configuration of the hemostasis valve in one embodiment of the present disclosure will be described below with reference to FIGs. 5 to 7A and 7B.

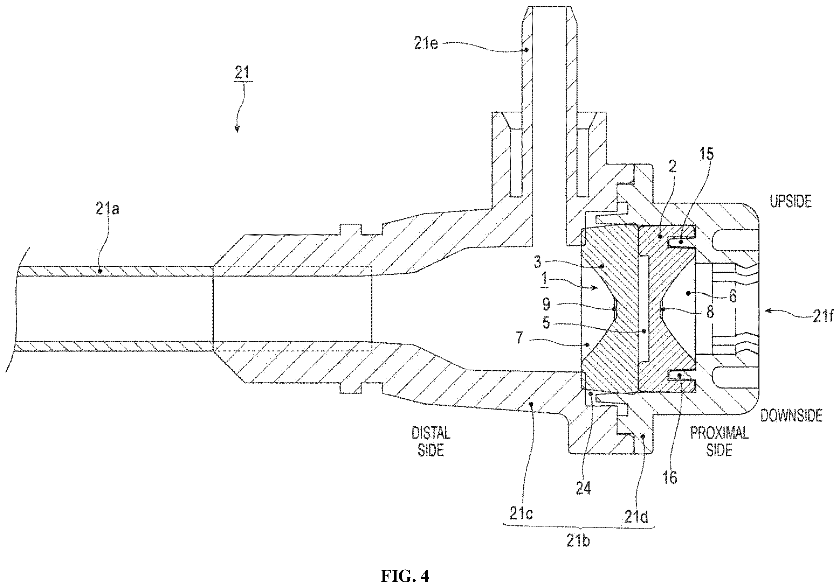

The hemostasis valve 1 shown in FIGs. 4 and 5 is a valve that is embedded into the valve container 24 in the sheath hub (valve housing) 21b of the sheath 21. Through the hemostasis valve 1, a tubular introduction member such as a dilator or a catheter is inserted. The hemostasis valve 1 has a double structure, which is expected to be more effective in stopping bleeding than a single valve.

As shown in FIGs. 4 and 5, the hemostasis valve 1 includes a first partition wall 2 in a proximal side, a second partition wall 3 in a distal side that is provided adjacently to the first partition wall 2, and a lubricant storage 5 that is provided between the first partition wall 2 and the second partition wall 3, the lubricant storage 5 having capability of storing lubricant.

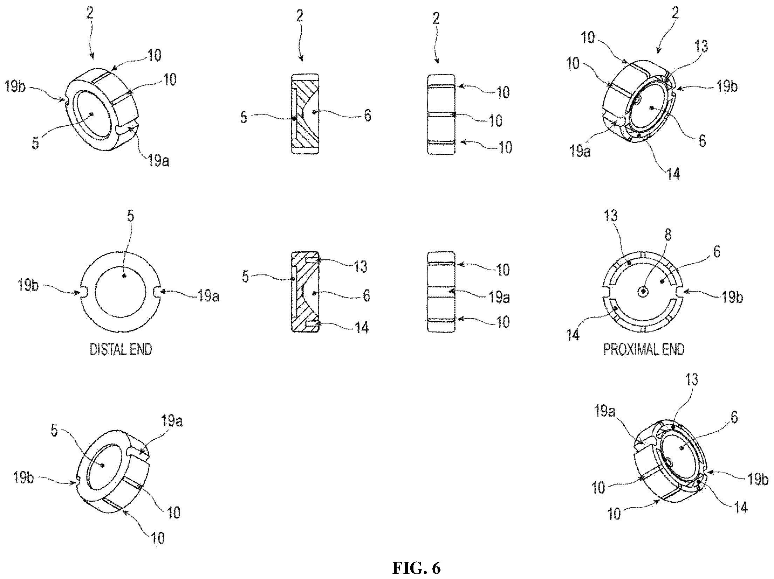

The lubricant storage 5 consists of a flat cylindrical concave formed on the inner (inside) face of the first partition wall 2 in the central axial direction (see FIGs. 6, 7A, and 7B).

The hemostasis valve 1 of this embodiment, "adjacent" means" being in direct contact with each other side by side.

The material of the first partition wall 2 and the second partition wall 3 is preferably made of a flexible and highly elastic polymeric material such as natural rubber, isoprene rubber, silicone rubber, or butadiene rubber.

As the lubricant, for example, a liquid lubricant can be used. Examples of the liquid lubricant include a lubricating oil, more specifically, a silicone oil. As the lubricant, a semi-solid lubricant such as a grease or a compound can also be used as lubricant. As the lubricant, for example, a solid lubricant such as molybdenum disulfide and graphite added to a liquid lubricant such as silicone oil can be used.

The configuration of the hemostasis valve 1 of the present disclosure has the following effects.

However, since a lubricant storage 5 that has capability of storing lubricant is provided between the first partition wall 2 and the second partition wall 3, the lubricant will never drip down due to gravity while a medical device 20 provided with the hemostasis valve 1 is being used or during the period from manufacture to use of the valve. Since the lubricant does not drip in this manner, the amount of the lubricant can be increased.

Therefore, the configuration of the hemostasis valve 1 of this embodiment has a structure that does not allow lubricant to drip down and that reliably enables a balance between improved sealing performance (prevention of air draw-in) and improved operability (slidability).

More details are explained below. As shown in FIGs. 1 to 4, the sheath hub (valve housing) 21b of the sheath 21 consists of a hub body 21c and a cap 21d. From the proximal side of the hub body 21c to the distal side of the cap 21d, the valve container 24 extends. The cap 21d is fixed to the hub body 21c by, for example, adhesive.

As shown in FIGs. 4 and 5, a first conical concave 6 and a second conical concave 7 are formed on the outer (outside) faces of the first partition wall 2 and the second partition wall 3 in the central axial direction, respectively. The inner ends of the first conical concave 6 and the second conical concave 7 are provided with centering holes (guide holes) 8, 9, respectively. A slit (not shown) of a Y-shape, a cross-shape, etc., cut from the centering hole 8 through the first partition wall 2. A slit (not shown) of a Y-shape, a cross-shape, etc., cut from the centering hole 9 through the second partition wall 3.

The first conical concave 6 formed in the first partition wall 2 functions as an inviting sloping face for insertion of a tubular introduction member such as a dilator or a catheter. The first conical concave 6 and the second conical concave 7 and the centering holes 8, 9 are coated with fluorine. As described above, the lubricant storage 5 consists of a flat cylindrical concave (with a diameter of approximately 5 mm and a depth of approximately 0.5 mm) formed on the inner (inside) face of the first partition wall 2 in the central axial direction. In other words, the lubricant storage 5 is formed by reducing the thickness of the first partition wall 2.

As shown in FIGs. 6, 7A, and 7B, on the upper and the lower arc faces of the outer circumferential face of the first partition wall 2, three grooves 10 for adhesive along the central axial direction are formed at regular intervals in the circumferential direction. Although not shown in the drawings, the same kind of grooves for adhesive are also formed on the outer circumferential face of the second partition wall 3.

The first partition wall 2 and the second partition wall 3 are embedded into the valve container 24 in the sheath hub (valve housing) 21b of the sheath 21 after adhesive is applied to the outer face. Since the outer diameters of the first partition wall 2 and the second partition wall 3 are larger than the inner diameter of the valve container 24 (reversed dimensions), most of the adhesive may be scraped off during the embedding. This may reduce the adhesive force between the first partition wall 2 and the second partition wall 3 and the valve container 24.

However, according to the configuration of the hemostasis valve 1 (the first partition wall 2 and the second partition wall 3), when the adhesive is applied, the adhesive is filled into the groove 10, etc., for the adhesive formed on the outer circumferential face of the first partition wall 2 and the second partition wall 3, and the adhesive will never be scraped off during the embedding. As the result, the decrease in adhesive force between the first partition wall 2 and the second partition wall 3 and the valve container 24 can be minimized.

The reversed dimensions between the outer diameters of the first partition wall 2 and the second partition wall 3 and the inner diameter of the valve container 24 allow the hemostasis valve 1 (the first partition wall 2 and the second partition wall 3) to be tightened inward when the hemostasis valve 1 is embedded into the valve container 24, thereby producing sealing effect of the slit in the center.

The distance L between the inner ends of the first conical concave 6 and the second conical concave 7 is set at 1.2 mm to 10.0 mm (see FIG. 5). According to this configuration, it is possible to improve sealing performance by distance to further ensure that air is not drawn in.

As shown in FIGs. 4 to 7A and 7B, upper and lower semicircular position fixing holes 13, 14 are formed in a pair on the outer edge of the outer face (outside) of the first partition wall 2 in the central axial direction. On the proximal end face of the valve container 24 in the cap 21d which composes the sheath hub (valve housing) 21b, upper and lower semicircular convex threads 15, 16 are provided in a pair, respectively, in the central axial direction, which fit into the position fixing holes 13, 14. The hemostasis valve 1 is embedded into the valve container 24 after the first partition wall 2 is first accommodated in the valve container 24 in the cap 21d, the second partition wall 3 is accommodated in the valve container 24, and then the cap 21d is fixed to the hub body 21c with, for example, adhesive. In this case, the first partition wall 2 has the convex threads 15, 16 on the valve container 24 side embedded in the position-fixing holes 13, 14, and the second partition wall 3 is pressed toward the first partition wall 2 by the distal end face of the valve container 24 in the central axial direction.

According to this configuration, when the hemostasis valve 1 (the first partition wall 2 and the second partition wall 3) is embedded into the valve container 24 in the sheath hub (valve housing) 21b of the sheath 21, the convex threads 15, 16 of the valve container 24 is embedded into the position fixing holes 13, 14 of the first partition wall 2, and the second partition wall 3 is pressed toward the first partition wall 2. As the result, the first partition wall 2 and the second partition wall 3 can be avoided from shifting (moving) around the central axis. Therefore, the appropriate relative position of the first partition wall 2 and the second partition wall 3 around the central axis can be secured, which can further improve operability (slidability) when a tubular introduction member such as a dilator or a catheter is inserted and removed. In addition, a slit of a Y shape, a cross shape, etc., can be maintained in the appropriate position to keep sealing performance.

When the adhesive is applied to the outer circumferential face of the first partition wall 2, the adhesive also runs around the inside of the position fixing holes 13, 14, increasing the adhesive area and strengthening the fixation force of the first partition wall 2 to the valve container 24 in the sheath hub (valve housing) 21b.

As shown in FIGs. 6, 7A, and 7B, on the outer circumferential face of the first partition wall 2, grooves 19a, 19b for directional control are formed in a pair on the right and the left sides, respectively, along the central axial direction. Although not shown in FIG. 4, in the valve container 24 in the cap 21d which composes the sheath hub (valve housing) 21b, a pair of straight convex threads are provided on the right and the left sides, which are inserted into the grooves 19a, 19b for directional control. Although not shown in the drawings, on the outer circumference of the second partition wall 3, a pair of grooves for directional control are formed on the right and the left sides, respectively, into which the pair of straight convex threads are inserted on the right and the left sides, respectively.

According to this configuration, when the hemostasis valve 1 (the first partition wall 2 and the second partition wall 3) is embedded into the valve container 24 in the sheath hub (valve housing) 21b of the sheath 21, the pair of convex threads are inserted into the pair of grooves 19a and 19b, etc., for directional control, so that the first partition wall 2 and the second partition wall 3 can be prevented from or restrained from being shifted relatively around the central axis. As the result, if the slits cut into the first partition wall 2 and the second partition wall 3 are, for example, cross-shaped (cross-cut), the slits become "*" shaped when the first partition wall 2 and the second partition wall 3 are superimposed on each other. This compensates for the cut sides (weak points) when a thin tubular introduction member is inserted so that sealing performance can be improved.

In addition, the medical device 20 shown in FIGs. 1 to 4 is provided with the above-mentioned hemostasis valve 1 (the first and the second hemostasis valve 2, 3) to make it possible to exert the action and effect caused by the above-mentioned hemostasis valve 1 (the first partition wall 2 and the second partition wall 3).

### How to use medical device

The use of the medical device in one embodiment of the present disclosure will be briefly explained below.

The medical device 20 of this embodiment is used integrally with a dilator (not shown) for example, when a cardiac catheter is inserted from a blood vessel (vena cava) into the left atrium.

First, heparin-saline solution (saline) is injected into the sheath tube 21a through a three-way stopcock before the sheath tube 21a is inserted into the blood vessel. After securing access to the femoral vein, the guide wire is inserted into the superior vena cava, and the dilator and the sheath tube 21a are inserted along the guide wire. When the tip of the dilator reaches the superior vena cava, the guide wire is removed, and the septal puncture needle is inserted into the sheath tube 21a. Then, the septal puncture needle is used to puncture the atrial septum, the dilator, and the sheath tube 21a are advanced into the left atrium, the dilator is withdrawn, and a cardiac catheter is inserted and placed at the target site.

In this case, if necessary, the rotating operation part (dial) 23 of the handle 22 is rotated to deflect the tip of the sheath tube 21a and adjust the indwelling position of the cardiac catheter.

In the medical device 20 of this embodiment, the hemostasis valve 1 is used as a hemostasis valve, which includes a first partition wall 2 in a proximal side, a second partition wall 3 in a distal side that is provided adjacently to the first partition wall 2, and a lubricant storage 5 that is provided between the first partition wall 2 and the second partition wall 3, the lubricant having capability of storing lubricant (see FIGs. 1, 2 and 4), so that the slidability of a dilator, a cardiac catheter, etc., can be maintained for a longer period. This also improves the sealing performance of the hemostasis valve 1 and avoids the risk of air draw-in.

In this embodiment, the case in which the valve is a hemostasis valve 1 for prevention of blood leak is used as an example. However, the valve of the present disclosure is not necessarily limited to hemostasis valves. The valve only has to be a valve through which a tubular introduction member is inserted.

In this embodiment, the case in which the tube member is an introducer sheath 21 used for, for example, inserting a cardiac catheter from a blood vessel (vena cava) into the left atrium is also described as an example. However, the present disclosure is not necessarily limited to such a configuration. The tube member may be any other tube member such as a catheter or an endoscopic tube as long as it is flexible.

In this embodiment, the case in which grooves 10, etc. for adhesive along the central axial direction are formed on the outer circumferential face of the first partition wall 2 and the second partition wall 3, respectively, is described as an example. However, the present disclosure is not necessarily limited to such a configuration. The grooves for adhesive need only be formed on at least one of the first partition wall 2 and the second partition wall 3. The grooves for adhesive may be formed, for example, along the circumferential direction of the outer circumferential face of the first partition wall 2 and/or the second partition wall 3.

In this embodiment, the hemostasis valve 1 is described using the example of a hemostasis valve 1 with only the first conical concave 6 and the second conical concave 7 formed on the outer (outside) face of the first partition wall 2 and the second partition wall 3, respectively, in the central axial direction. However, the present disclosure is not necessarily limited to such a configuration.

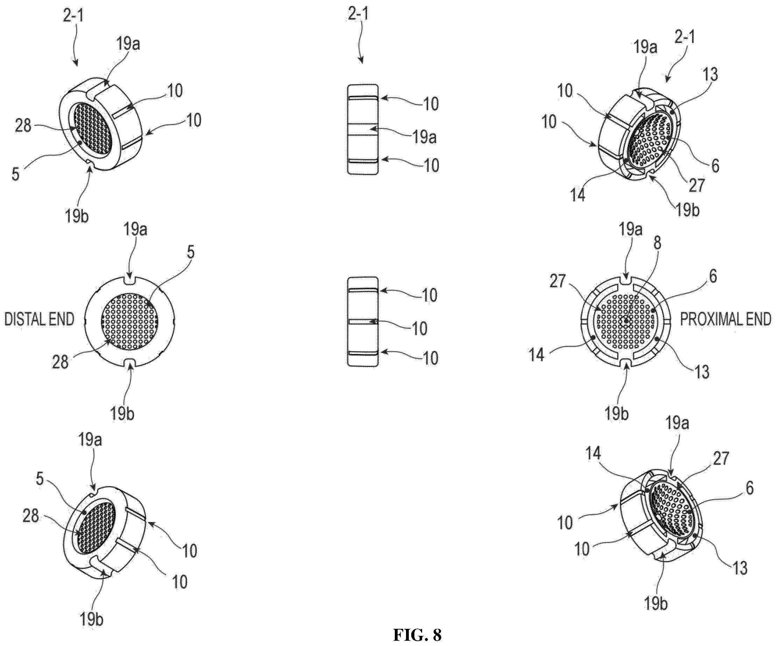

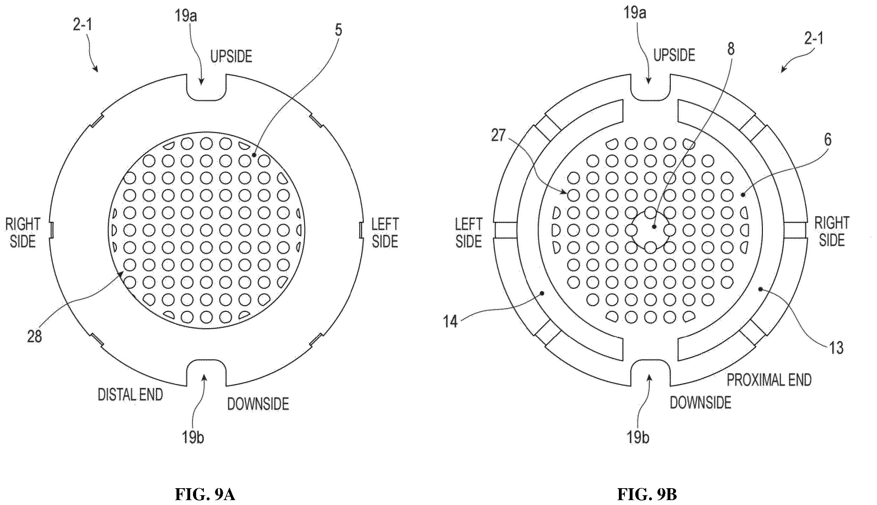

As shown in FIGs. 8 and 9, a number of dimple-shaped lubricant reservoirs 27, 28 may be formed on the outer and the inner (inside) faces of the first partition wall 2-1 in the central axial direction. Although not shown in the drawings, a number of dimple-shaped lubricant reservoirs may also be formed on the outer and the inner faces of the second partition wall 3 in the central axial direction. A lubricant reservoir only has to be formed on at least the outer face of the first partition wall 2 and/or the second partition wall 3 in the central axial direction. In addition to a dimple-shaped lubricant reservoir, a groove-shaped lubricant reservoir can also be formed, for example.

According to this configuration, even when a lubricant is applied on at least the outer face of the first partition wall 2 and/or the second partition wall 3 in the central axial direction, the lubricant remains in the lubricant reservoirs 27, 28. As the result, the lubricant is less likely to drip down due to gravity during the period of manufacture to use of the valve or while a medical device 20 provided with the hemostasis valve 1 is being used. Therefore, the lubricant applied to at least the outer face of the first partition wall 2 and/or the second partition wall 3 in the central axial direction also helps to enable the operability (slidability) and the sealing performance (prevention of air draw-in) to be further improved when a tubular introduction member such as a dilator or a cardiac catheter is inserted and removed.

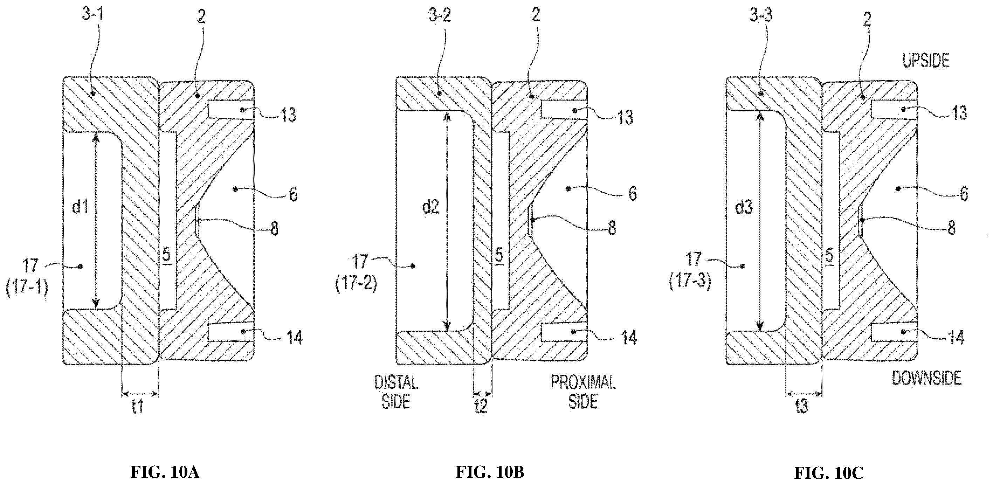

In this embodiment, the case in which the second conical concave 7 is formed on the outer (outside) face of the second partition wall 3 in the central axial direction is described as an example. However, the present disclosure is not necessarily limited to such a configuration. As shown in FIGs. 10A, 10B, and 10C, a tub-shaped concave 17 with a flat bottom may be formed on the outer (outside) face of the second partition walls 3-1, 3-2, 3-3 in the central axial direction. In FIG. 10A, the diameter d1 of the tub-shaped concave 17-1 is approximately 5 mm and the thickness t1 of the valve portion of the second partition wall 3-1 is approximately 1 mm. In FIG. 10B, the diameter d2 of the tub-shaped concave 17-2 is approximately 6.5 mm and the thickness t2 of the valve portion of the second partition wall 3-2 is approximately 0.5 mm. In FIG. 10C, the diameter d3 of the tub-shaped concave 17-3 is approximately 6.5 mm and the thickness t3 of the valve portion of the second partition wall 3-3 is approximately 1 mm.

According to this configuration, the valve portions of the second partition walls 3-1 to 3-3 can be easily opened.

In this embodiment, the case in which the lubricant storage 5 consists of a flat cylindrical concave formed on the inner (inside) face of the first partition wall 2 in the central axial direction is described as an example. However, the present disclosure is not necessarily limited to such a configuration.

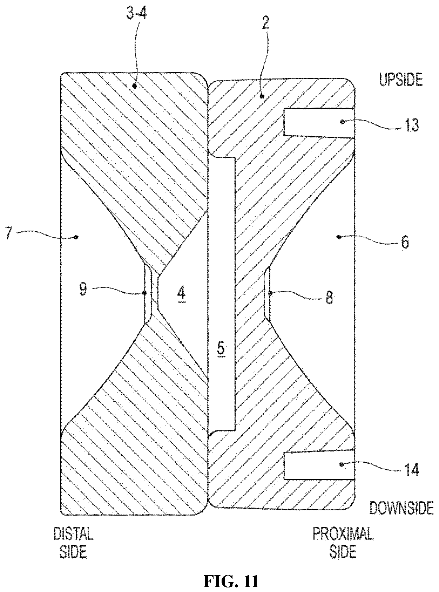

As shown in FIG. 11, a third conical concave 4 may be further formed on the inner (inside) face of the second partition wall 3-4 in the central axial direction as part of the lubricant storage. According to this configuration, it is possible to restrict, for example, a pigtail- or ring-shaped catheter from not advancing straight and from straying into the lubricant storage when such a catheter is inserted. According to this configuration, the amount of lubricant also can be further increased to make it possible to maintain the slidability of a dilator, a cardiac catheter, etc. for an even longer period.

Only the third conical concave 4 may also compose the lubricant storage. As described above, the lubricant storage 5 can be variously shaped and positioned. For example, the lubricant storage 5 may consist of a flat cylindrical concave formed on the inner (inside) face of the second partition wall 3 in the central axial direction. The lubricant storage 5 may consist of a flat cylindrical concave formed on the inner (inside) faces of the partition walls 2, 3 in the central axial direction.

In this embodiment, the case in which the semicircular position fixing holes 13, 14 are formed on the outer edge of the (outside) face (proximal face) of the first partition wall 2 in the central axial direction is described as an example. However, the present disclosure is not necessarily limited to such a configuration.

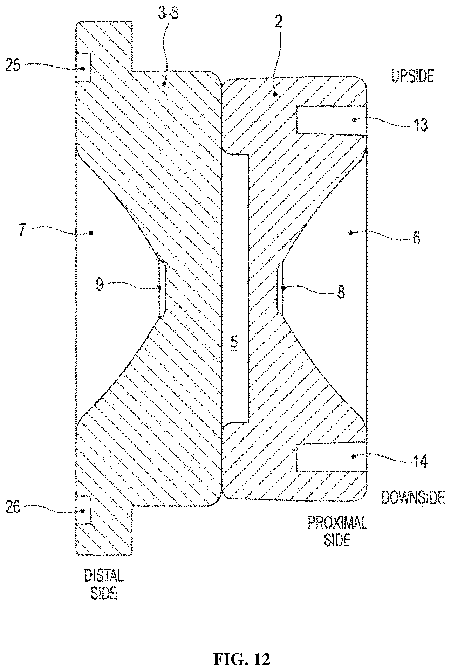

As shown in FIG. 12, the semicircular position fixing holes 25, 26 may be formed on the outer edge of the outer (outside) face (distal face) of the second partition wall 3-5 in the central axial direction. The position fixing holes may be formed only in the second partition wall.

In addition to the position fixing holes 13, 14, for example, a position fixing hole consisting of multiple circular holes may also be formed.

### Description of reference numerals

1: hemostasis valve, 2: first partition wall, 3, 3-1, 3-2, 3-3, 3-4, 3-5: second partition wall, 5: lubricant storage, 6: first conical concave, 7: second conical concave, 8, 9: centering hole (guide hole), 10: groove for adhesive, 13,14,25,26: position fixing hole, 15,16: convex thread, 17-1,17-2,17-3: tub-shaped convex, 19a,19b: groove for directional control, 20: medical device, 21: introducer sheath, 21a: sheath tube, 21b: sheath hub (valve housing), 21c: hub body, 21d: cap, 21e side: port, 21f: insertion port, 22: handle, 23: rotating operation part (dial), 24: valve container, 27, 28: lubricant reservoir.

## Claims

1. A valve through which a tubular introduction member is inserted, comprising:
a first partition wall in a proximal side;
a second partition wall in a distal side that is provided adjacently to the first partition wall; and
a lubricant storage that is provided between the first partition wall and the second partition wall, the lubricant storage having capability of storing lubricant.

2. The valve according to claim 1, further comprising a groove for an adhesive that is formed on the outer circumferential face of the first partition wall and/or the second partition wall.

3. The valve according to claim 1, further comprising a lubricant reservoir that is formed on at least the outer (outside) face of the first partition wall and/or the second partition wall in the central axial direction.

4. The valve according to claim 1, further comprising a position fixing hole that is formed on the outer edge of the outer face of the first partition wall and/or the second partition wall in the central axial direction.

5. The valve according to claim 1, further comprising a tub-shaped concave with a flat bottom that is formed on the central axially outer (outside) face of the second partition wall in the central axial direction.

6. The valve according to claim 1, further comprising a first and a second conical concaves are formed on the outer (outside) face of the first partition wall and the second partition wall in the central axial direction, respectively, and the distance between the inner ends of the first and the second conical concaves is set at 1.2 to 10.0 mm.

7. The valve according to claim 1, wherein the lubricant storage includes at least a third conical concave formed on the inner (inside) face of the second partition wall in the central axial direction.

8. A medical device comprising the valve according to any one of claims 1 to 7.
